# EUROPÄISCHE PATENTSCHRIFT

(11) **EP 2 300 061 B1**
(45) Veröffentlichungstag und Bekanntmachung des Hinweises auf die Patenterteilung: **18.06.2014**
(21) Anmeldenummer: 09780416.5
(22) Anmeldetag: 10.07.2009
(51) Int. Cl.: A61L 15/22, A61L 15/60, C08J 3/24, C08K 3/00, C08L 101/14

(54) **VERFAHREN ZUR OBERFLÄCHENNACHVERNETZUNG WASSERABSORBIERENDER POLYMERPARTIKEL**
METHOD FOR THE SURFACE POST-CROSSLINKING OF WATER ABSORBING POLYMER PARTICLES
PROCÉDÉ DE POST-RÉTICULATION DE SURFACE DE PARTICULES DE POLYMÈRE HYDROPHILES

(30) Priorität: 11.07.2008 EP 08160175
(43) Veröffentlichungstag der Anmeldung: 30.03.2011
(73) Patentinhaber: BASF SE, 67056 Ludwigshafen (DE)
(72) Erfinder: FUNK, Rüdiger, 65527 Niedernhausen (DE); HEIDE, Wilfried, 67251 Freinsheim (DE); STUEVEN, Uwe, 65812 Bad Soden (DE)
(86) Internationale Anmeldenummer: PCT/EP2009/058798
(87) Internationale Veröffentlichungsnummer: WO 2010/004020

(56) Entgegenhaltungen:
- WO-A-91/18042
- WO-A-2004/069404
- WO-A-2004/069936
- WO-A-2005/092955
- WO-A-2009/005114
- WO-A1-2009/041731
- DE-A1- 10 130 021
- DE-A1-102005 004 285
- US-A- 5 462 972
- US-A1- 2008 221 229
- US-B1- 6 350 710

## Beschreibung

Die vorliegende Erfindung betrifft ein Verfahren zur Herstellung wasserabsorbierender Polymerpartikel, wobei die wasserabsorbierenden Polymerpartikel mit ungesättigten Fettsäuren oder deren Derivaten oberflächennachvernetzt werden.

Wasserabsorbierende Polymere werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet und bestehen aus hydrophilen Polymeren, die so stark vernetzt sind, dass sie nicht mehr löslich sind.

Die Herstellung der wasserabsorbierenden Polymere wird beispielsweise in der Monographie "Modern Superabsorbent Polymer Technology", F.L. Buchholz und A.T. Graham, Wiley-VCH, 1998, Seiten 71 bis 103, und in Ullmann's Encyclopedia of Industrial Chemistry, 6. Auflage, Band 35, Seiten 73 bis 93, beschrieben.

Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm² (AUL0.3psi) durchläuft ein Maximum.

Zur Verbesserung der Anwendungseigenschaften in der Windel, wie Permeabilität des gequollenen Gelbetts (SFC) und Absorption unter einem Druck von 49,2 g/cm² (AUL0.7psi) oder mehr, werden wasserabsorbierende Polymerpartikel üblicherweise oberflächennachvernetzt. Dadurch steigt nur der Vernetzungsgrad der Partikeloberfläche, wodurch die Zentrifugenretentionskapazität (CRC) und die Absorption unter einem Druck von 49,2 g/cm² (AUL0.7psi) zumindest teilweise entkoppelt werden können. Zur Oberflächennachvernetzung werden vorzugsweise getrocknete, gemahlene und abgesiebte wasserabsorbierende Polymerpartikel (Grundpolymer) mit einem Oberflächennachvernetzer beschichtet und thermisch oberflächennachvernetzt.

Bei Verwendung di- und polyfunktioneller Epoxide kann bei besonders niedrigen Temperaturen thermisch oberflächennachvernetzt werden. So genügen für die Oberflächennachvernetzung mit Ethylenglykoldiglycidylether bereits 30 Minuten bei 140°C. EP 0 668 080 A2 und 0 780 424 A1 beschreiben Verfahren zur Umsatzerhöhung der eingesetzten Epoxide, da im Endprodukt verbleibende Restmengen der zur Oberflächennachvernetzung eingesetzten Epoxide toxikologisch bedenklich sein sollen.

Aufgabe der vorliegenden Erfindung war die Bereitstellung eines Verfahrens zur thermischen Oberflächennachvernetzung wasserabsorbierender Polymerpartikel, wobei die thermische Oberflächennachvemetzung bei niedrigen Temperaturen durchgeführt werden und trotzdem auf die Verwendung toxikologisch bedenklicher Oberflächennachvernetzer verzichtet werden kann.

Gelöst wurde die Aufgabe durch ein Verfahren zur Herstellung oberflächennachvernetzter wasserabsorbierender Polymerpartikel, wobei die wasserabsorbierenden Polymerpartikel durch Polymerisation einer Monomerlösung oder-suspension, enthaltend
a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
erhalten werden, mindestens ein Oberflächennachvernetzer auf die wasserabsorbierenden Polymerpartikel aufgebracht wird und die wasserabsorbierenden Polymerpartikel thermisch oberflächennachvernetzt werden, dadurch gekennzeichnet, dass der Oberflächennachvernetzer mindestens eine ethylenisch ungesättigte Carbonsäure mit mindestens 10 Kohlenstoffatomen, deren Salz, Ester und/oder Epoxidierungsprodukt ist.

Die ethylenisch ungesättigten Carbonsäuren, deren Salze, Ester und/oder Epoxidierungsprodukte haben vorzugsweise mindestens 12, besonders bevorzugt mindestens 14, ganz besonders bevorzugt mindestens 16, sowie üblicherweise weniger als 30, Kohlenstoffatome.

Die ethylenisch ungesättigten Carbonsäuren, deren Salze und/oder Ester haben mindestens zwei, besonders bevorzugt mindestens drei, ganz besonders bevorzugt mindestes 4, Kohlenstoff-Kohlenstoff-Doppelbindungen (ethylenisch ungesättigte Gruppen).

Vorteilhaft werden ethylenisch ungesättigten Carbonsäuren mit benachbarten Kohlenstoff-Kohlenstoff-Doppelbindungen, deren Salze und/oder Ester verwendet. Benachbarte Kohlenstoff-Kohlenstoff-Doppelbindungen sind zwei Kohlenstoff-Kohlenstoff-Doppelbindungen, die durch genau ein Kohlenstoffatom, vorzugsweise eine Methylen-Gruppe, getrennt sind, wie beispielsweise in Linolsäure und Linolensäure. Diese ethylenisch ungesättigten Carbonsäuren, deren Salze und/oder Ester sind besonders reaktiv.

In einer bevorzugten Ausführungsform der vorliegenden Erfindung enthält die ethylenisch ungesättigte Carbonsäure, deren Salz und/oder Ester zusätzlich mindestens eine Epoxid-Gruppe, vorzugsweise mindestens eine zur Kohlenstoff-Kohlenstoff-Doppelbindung benachbarte Epoxid-Gruppe, wie beispielsweise in Vernolsäure.

Die im erfindungsgemäßen Verfahren einsetzbaren ethylenisch ungesättigten Carbonsäuren, deren Salze und/oder Ester werden vorzugsweise aus nachwachsenden Rohstoffen gewonnen. Geeignete nachwachsende Rohstoffe sind pflanzliche und/oder tierische Öle, wie Butterfett, Rapsöl, Rizinusöl, Hühnerfett, Zitronenkernöl, Kakaobutter, Kokosöl, Lebertran, Maiskeimöl, Baumwollsaatöl, Schweinefett, Leinöl, Heringstran, Olivenöl, Palmöl, Palmkernöl, Erdnussöl, Traubenkernöl, Reisöl, Distelöl, Sesamöl, Sojaöl, Sonnenblumenöl, Rindertalg und China-Holzöl. Die in nachwachsenden Rohstoffen vorkommenden ethylenisch ungesättigten Carbonsäuren, deren Salze und/oder Ester (ungesättigte Fettsäuren) sind bevorzugt.

Die ethylenisch ungesättigten Carbonsäuren, deren Salze und/oder Estern können auch zusammen mit anderen Carbonsäuren, deren Salzen und/oder Estern eingesetzt werden, wobei der Anteil an ethylenisch ungesättigter Carbonsäure, deren Salz und/oder Ester im Gemisch vorzugsweise mindestens 30 Gew.-%, besonders bevorzugt mindestens 40 Gew.-%, ganz besonders bevorzugt mindestens 50 Gew.-%, beträgt, d.h., die beispielsweise aus tierischen und/oder pflanzlichen Ölen gewonnenen Fettsäuregemische können, sofern der Anteil an ungesättigten Fettsäuren hoch genug ist, direkt eingesetzt werden.

Ganz besonders bevorzugt werden Leinöl und/oder Vernoniaöl und die aus Leinöl und/oder Vernoniaöl gewonnen Fettsäuregemische verwendet. Die in Leinöl enthaltenen Carbonsäuren sind beispielsweise Stearinsäure (ca. 1 bis 4 Gew.-%), Palmitinsäure (ca. 4 bis 8 Gew.-%), Linolsäure (ca. 10 bis 30 Gew.-%), Ölsäure (ca. 15 bis 30 Gew.-%) und Linolensäure (ca. 40 bis 68 Gew.-%).

Unter Estern der ethylenisch ungesättigten Carbonsäuren werden vorzugsweise Fette und Öle verstanden, d.h. Fettsäureester des Glyzerins.

Werden die ethylenisch ungesättigten Carbonsäuren, deren Salze und/oder Ester als Oberflächennachvernetzer eingesetzt, so wird die Oberflächennachvernetzung vorteilhaft in Gegenwart von Sauerstoff durchgeführt. Der Sauerstoffpartialdruck beträgt während der thermischen Oberflächennachvernetzung in dem verwendeten Apparat vorzugsweise mindestens 10 mbar, besonders bevorzugt mindestens 50 mbar, ganz besonders bevorzugt mindestens 100 mbar. Die thermische Oberflächennachvernetzung kann beispielsweise unter Luft durchgeführt werden. Sauerstoff reagiert mit den ethylenisch ungesättigten Carbonsäuren zu Hydroperoxiden, die zu radikalischen Vernetzungen führen. Diese Vernetzung ungesättigter Fettsäuren wird auch als "Trocknung" bezeichnet.

Die "Trocknung" der ethylenisch ungesättigten Carbonsäure ermöglicht eine thermische Oberflächennachvernetzung bei deutlich niedrigeren Temperaturen, vorzugsweise 50 bis 150°C, besonders bevorzugt 60 bis 130°C, ganz besonders bevorzugt 70 bis 100°C. Selbstverständlich kann die Oberflächennachvernetzung aber auch bei höheren Temperaturen durchgeführt werden.

In einer ganz besonders bevorzugten Ausführungsform der vorliegenden Erfindung werden die Kohlenstoff-Kohlenstoff-Doppelbindungen der ethylenisch ungesättigten Carbonsäuren vor der Oberflächennachvernetzung zumindest teilweise in die entsprechenden Epoxid-Gruppen umgewandelt. Die Epoxidierung von Kohtenstoff-Kohlenstoff-Doppelbindungen wird beispielsweise in Organikum - Organisch-chemisches Grundpraktikum, 16. Auflage, VEB Deutscher Verlag der Wissenschaften, Berlin, 1986, Seiten 257 bis 261, beschrieben.

In einer weiteren ganz besonders bevorzugten Ausführungsform der vorliegenden Erfindung werden die Kohlenstoff-Kohlenstoff-Doppelbindungen von Estern der ethylenisch ungesättigten Carbonsäuren vor der Oberflächennachvernetzung zumindest teilweise in die entsprechenden Epoxid-Gruppen umgewandelt. Ganz besonders bevorzugt werden epoxidierte pflanzliche und/oder tierische Öle als Oberflächennachvernetzer eingesetzt. Die Herstellung epoxidierter pflanzlicher und/oder tierischer Öle wird beispielsweise in J. Polym. Sci. Part A: Polym. Chem. Jahrgang 2006, Band 44, Seiten 6717 bis 6727, und Eur. Polym. J. Jahrgang 2005, Band 41, Seite 231 bis 237, beschrieben.

Werden die Epoxidierungsprodukte als Oberflächennachvernetzer eingesetzt, so wird die Oberflächennachvernetzung vorteilhaft in Abwesenheit von Sauerstoff durchgeführt. Der Sauerstoffpartialdruck beträgt daher während der thermischen Oberflächennachvernetzung in dem verwendeten Apparat vorzugsweise weniger als 100 mbar, besonders bevorzugt weniger als 50 mbar, ganz besonders bevorzugt weniger als 10 mbar.

Die thermische Oberflächennachvernetzung kann bei deutlich niedrigeren Temperaturen, vorzugsweise 50 bis 150°C, besonders bevorzugt 60 bis 130°C, ganz besonders bevorzugt 70 bis 100°C. Selbstverständlich kann die Oberflächennachvernetzung aber auch bei höheren Temperaturen durchgeführt werden.

Im Folgenden wird die Herstellung der, üblicherweise wasserunlöslichen, wasserabsorbierenden Polymerpartikel näher erläutert.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/1104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraalloxyethan, Methylenbismethacrylamid, 15-fach ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3-bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 g/cm² (AUL0.3psi) durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; DE) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter. Es ist aber auch möglich eine wässrige Monomerlösung zu vertropfen und die erzeugten Tropfen in einem erwärmten Trägergasstrom zu polymerisieren. Hierbei können die Verfahrensschritte Polymerisation und Trocknung zusammengefasst werden, wie in WO 2008/040715 A2 und WO 2008/052971 A1 beschrieben.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA (European Disposables and Nonwovens Association) empfohlenen Testmethode Nr. WSP 230.2-05 "Moisture Content" bestimmt wird. Bei einer zu hohen Restfeuchte weist das getrocknete Polymergel eine zu niedrige Glasübergangstemperatur T_{g} auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 µm, besonders bevorzugt von 250 bis 600 µm, ganz besonders von 300 bis 500 µm. Die mittlere Partikelgröße der Produktfraktion kann mittels der von der EDANA (European Disposables and Nonwovens Association) empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 µm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Die geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich zu kleine Polymerpartikel nach der Oberflächennachvernetzung abzutrennen.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 µm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel werden zur weiteren Verbesserung der Eigenschaften mit ethylenisch ungesättigten Carbonsäuren oder deren Derivaten oberflächennachvernetzt.

Die Menge an ethylenisch ungesättigter Carbonsäure oder deren Derivat beträgt 0,02 bis 2 Gew.-%, besonders bevorzugt 0,02 bis 1 Gew.-%, ganz besonders bevorzugt 0,05 bis 0,2 Gew.-%, jeweils bezogen auf die wasserabsorbierenden Polymerpartikel.

Die ethylenisch ungesättigten Carbonsäuren und deren Derivate werden vorzugsweise verdünnt eingesetzt, d.h. gelöst oder dispergiert in einem geeigneten Lösungsmittel, beispielsweise Wasser, Methanol, Isopropanol, 1,3-Propandiol, Ethylenglykol, Propylenglykol, Polyethylenglykol und/oder Polypropylenglykol. Um die Dispergierung in Wasser und das Benetzungsverhalten der Dispersion zu verbessern können statt der ethylenisch ungesättigten Carbonsäuren auch deren Salze, beispielsweise die Natriumsalze, eingesetzt werden.

Zusätzlich können noch weitere Oberflächennachvernetzer eingesetzt werden. Geeignete weitere Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete weitere Oberflächennachvernetzer beschrieben.

Bevorzugte weitere Oberflächennachvernetzer sind Ethylenkarbonat und Gemische aus Propylenglykol und 1,4-Butandiol.

Ganz besonders bevorzugte Oberflächennachvernetzer sind 2-Hydroxyethyloxazolidin-2-on, Oxazolidin-2-on und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die weiteren Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpar-tikel eingestellt werden.

Wird ausschließlich Wasser als Lösungsmittel für die weiteren Oberflächennachvernetzer verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung oder Dispersion des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung oder Dispersion des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; DE), Vrieco-Nauta Continuous Mixer (Hosokawa Micron BV; Doetinchem; NL), Processall Mixmill Mixer (Processall Incorporated; Cincinnati; US) und Schugi Flexomix® (Hosokawa Micron BV; Doetinchem; NL). Es ist aber auch möglich die Oberflächennachvernetzerlösung in einem Wirbelbett aufzusprühen.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; DE), Hosokawa Bepex® Disc Dryer (Hosokawa Micron GmbH; Leingarten; DE) und Nara Paddle Dryer (NARA Machinery Europe; Frechen; DE). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Werden zusätzlich zu den erfindungsgemäßen Oberflächennachvernetzern noch weitere Oberflächennachvernetzer eingesetzt, so kann es zweckmäßig sein aufgrund der möglicherweise geringeren Reaktivität dieser weiteren Oberflächennachvernetzer die thermische Oberflächennachvernetzung bei höheren Temperaturen durchzuführen, üblicherweise bei 100 bis 250°C, vorzugsweise bei 120 bis 220°C, besonders bevorzugt bei 130 bis 210°C, ganz besonders bevorzugt bei 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden. Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Die gemäß dem erfindungsgemäßen Verfahren hergestellten wasserabsorbierenden Polymerpartikel weisen einen Feuchtegehalt von vorzugsweise 0 bis 15 Gew.-%, besonders bevorzugt 0,2 bis 10 Gew.-%, ganz besonders bevorzugt 0,5 bis 8 Gew.-%, auf, wobei der Wassergehalt gemäß der von der EDANA (European Disposables and Nonwovens Association) empfohlenen Testmethode Nr. WSP 230.2-05 "Moisture Content" bestimmt wird.

Die gemäß dem erfindungsgemäßen Verfahren hergestellten wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA (European Disposables and Nonwovens Association) empfohlenen Testmethode Nr. WSP 241.2-05 "Centrifuge Retention Capacity" bestimmt.

Die gemäß dem erfindungsgemäßen Verfahren hergestellten wasserabsorbierenden Polymerpartikel weisen eine Absorption unter einem Druck von 49,2 g/cm² (AUL0.7psi) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 g/cm² (AUL0.7psi) der wasserabsorbierenden Polymerpartikel beträgt üblicherweise weniger als 35 g/g. Die Absorption unter einem Druck von 49,2 g/cm² wird analog der von der EDANA (European Disposables and Nonwovens Association) empfohlenen Testmethode Nr. WSP 242.2-05 "Absorption under Pressure" bestimmt, wobei statt eines Drucks von 21,0 g/cm² (AUL0.3psi) ein Druck von 49,2 g/cm² (AUL0.7psi) eingestellt wird.

Weitere Gegenstände der vorliegenden Erfindung sind wasserabsorbierende Polymerpartikel, erhältlich gemäß dem erfindungsgemäßen Verfahren, sowie Hygieneartikel, die diese wasserabsorbierenden Polymerpartikel enthalten.

### Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

### Zentrifugenretentionskapazität (Centrifuge Retention Capacity)

Die Zentrifugenretentionskapazität (CRC) wird gemäß der von der EDANA (European Disposables and Nonwovens Association) empfohlenen Testmethode Nr. WSP 241.2-05 "Centrifuge Retention Capacity" bestimmt.

### Absorption unter einem Druck von 21,0 g/cm² (Absorption under Pressure)

Die Absorption unter einem Druck von 21,0 g/cm² (AUL0.3psi) wird gemäß der von der EDANA (European Disposables and Nonwovens Association) empfohlenen Testmethode Nr. WSP 242.2-05 "Absorption under Pressure" bestimmt.

Die EDANA-Testmethoden sind beispielsweise erhältlich bei der European Disposables and Nonwovens Association, Avenue Eugene Plasky 157, B-1030 Brüssel, Belgien.

### Beispiele

### Beispiel 1 (Herstellung des Grundpolymers)

Durch kontinuierliches Mischen von entionisiertem Wasser, 50 gew.-%iger Natronlauge und Acrylsäure wurde eine Acrylsäure/Natriumacrylatlösung hergestellt, so dass der Neutralisationsgrad 72 mol-% entsprach. Der Feststoffgehalt der Monomerlösung betrug 40 Gew.-%.

Als mehrfach ethylenisch ungesättigter Vernetzer wurde 3-fach ethoxyliertes Glyzerintriacrylat verwendet. Die Einsatzmenge betrug 1,3 kg Vernetzer pro t Monomerlösung.

Zur Initiierung der radikalischen Polymerisation wurden pro t Monomerlösung 1 kg einer 0,25gew.-%igen wässriger Wasserstoffperoxidlösung, 1,5 kg einer 30gew.-%igen wässrigen Natriumperoxodisulfatlösung und 1 kg einer 1 gew.-%igen wässrigen Ascorbinsäurelösung eingesetzt.

Der Durchsatz der Monomerlösung betrug 18 t/h. Die Reaktionslösung hatte am Zulauf eine Temperatur von 30°C.

Die einzelnen Komponenten wurden in folgenden Mengen kontinuierlich in einen Reaktor vom Typ List Contikneter mit einem Volumen 6,3m³ (LIST AG, Arisdorf, CH) dosiert:

| | |
|---|---|
| 18 t/h | Monomerlösung |
| 23,4 kg/h | Polyethylenglykol-400-diacrylat |
| 45 kg/h | Wasserstoffperoxidlösung/Natriumperoxodisulfatlösung |
| 18 kg/h | Ascorbinsäurelösung |

Zwischen dem Zugabepunkt für den Vernetzer und den Zugabestellen für die Initiatoren wurde die Monomerlösung mit Stickstoff inertisiert.

Es fand nach ca. 50% der Verweilzeit zusätzlich eine Zudosierung von aus dem Herstellungsprozeß durch Mahlung und Siebung anfallendem Feinkorn (1000 kg/h) in den Reaktor statt. Die Verweilzeit der Reaktionsmischung im Reaktor betrug 15 Minuten.

Das erhaltene Produktgel wurde auf einen Bandtrockner aufgegeben. Auf dem Bandtrockner wurde das Polymergel kontinuierlich mit einem Luft/Gasgemisch umströmt und bei 175°C getrocknet. Die Verweilzeit im Bandtrockner betrug 37 Minuten.

Das getrocknete Polymergel wurde gemahlen und auf eine Partikeigrößenfraktion von 150 bis 850µm abgesiebt. Das so erhaltene Grundpolymer hatte eine Zentrifugenretentionskapazität (CRC) von 36,5 g/g und eine Absorption unter einem Druck von 21,0 g/cm² (AUL0.3psi) von 12,8 g/g.

### Beispiel 2 (Vergfeichsbeispiel)

Das in Beispiel 1 hergestellte Grundpolymer (200 g) wurde in einem Vakuumtrockenschrank (Heraeus VACUTHERM VT 6060M; Kendro Laboratory Products GmbH, DE) für 60 Minuten bei 150°C wärmebehandelt.

Das wärmebehandelte Grundpolymer hatte eine Zentrifugenretentionskapazität (CRC) von 38,8 g/g und eine Absorption unter einem Druck von 21,0 g/cm² (AUL0.3psi) von 13,9 g/g.

### Beispiel 3

Das in Beispiel 1 hergestellte Grundpolymer (200 g) wurde in einer Küchenmaschine (ProfiMixx 47; Robert Bosch GmbH; DE) bei Rührstufe 4 mittels einer Zweistoffdüse (0,2 bar Sprühdruck, N₂ , 8g/min) mit einer Oberflächennachvernetzerlösung, bestehend aus 1,0 g Leinöl und 7,0 g Isopropanol, besprüht. Die feuchten Polymerpartikel wurden mit einem Spatel nochmals homogenisiert und anschließend in einem Vakuumtrockenschrank (Heraeus VACUTHERM VT 6060M; Kendro Laboratory Products GmbH, DE) für 60 Minuten bei 150°C wärmebehandett.

Die nachvernetzten Polymerpartikel wurden über ein 850 µm Sieb schutzgesiebt und analysiert.

Das oberflächennachvernetzte Grundpolymer hatte eine Zentrifugenretentionskapazität (CRC) von 37,7 g/g und eine Absorption unter einem Druck von 21,0 g/cm² (AUL0.3psi) von 16,9 g/g.

### Beispiel 4

Es wurde Verfahren wie unter Beispiel 3, wobei eine Oberflächennachvemetzerlösung, bestehend aus 1,0 g Leinöl, 6,6 g Isopropanol und 0,4 g Wasser, eingesetzt wurde.

Das oberflächennachvernetzte Grundpolymer hatte eine Zentrifugenretentionskapazität (CRC) von 38,1 g/g und eine Absorption unter einem Druck von 21,0 g/cm² (AUL0.3psi) von 22,9 g/g.

## Patentansprüche

1. Verfahren zur Oberflächennachvernetzung wasserabsorbierender Polymerpartikel, wobei die wasserabsorbierenden Polymerpartikel durch Polymerisation einer Monomerlösung oder-suspension, enthaltend
a) mindestens ein ethylenisch ungesättigtes, säuregruppentragendes Monomer,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
erhalten werden, mindestens ein Oberflächennachvernetzer auf die wasserabsorbierenden Polymerpartikel aufgebracht wird und die wasserabsorbierenden Polymerpartikel thermisch oberflächennachvernetzt werden, **dadurch gekennzeichnet, dass** der Oberflächennachvernetzer mindestens eine ethylenisch ungesättigte Carbonsäure mit mindestens 10 Kohlenstoffatomen und mindestens zwei Kohlenstoff-Kohlenstoff-Doppelbindungen, deren Salz, Ester und/oder Epoxidierungsprodukt ist und die Menge an ethylenisch ungesättigter Carbonsäure mit mindestens 10 Kohlenstoffatomen und mindestens zwei Kohlenstoff-Kohlenstoff-Doppelbindungen, deren Salz, Ester und/oder Epoxidierungsprodukt, bezogen auf die wasserabsorbierenden Polymerpartikel, von 0,02 bis 2 Gew.-% beträgt.

2. Verfahren gemäß Anspruch 1, **dadurch gekennzeichnet, dass** die ethylenisch ungesättigte Carbonsäure, deren Salz oder Ester mindestens zwei benachbarte Kohlenstoff-Kohlenstoff-Doppelbindungen aufweist.

3. Verfahren gemäß einem der Ansprüche 1 oder 2, **dadurch gekennzeichnet, dass** die ethylenisch ungesättigte Carbonsäure, deren Salz oder Ester zusätzlich zur Kohlenstoff-Kohlenstoff-Doppelbindung mindestens eine Epoxid-Gruppe aufweist.

4. Verfahren gemäß einem der Ansprüche 1 bis 3, **dadurch gekennzeichnet, dass** die ethylenisch ungesättigte Carbonsäure, deren Salz oder Ester zusätzlich mindestens eine zur Kohlenstoff-Kohlenstoff-Doppelbindung benachbarte Epoxid-Gruppe aufweist.

5. Verfahren gemäß einem der Ansprüche 1 bis 4, **dadurch gekennzeichnet, dass** die ethylenisch ungesättigte Carbonsäure, deren Salz oder Ester Linolensäure, deren Salz oder Ester und/oder Vernolsäure, deren Salz oder Ester ist.

6. Verfahren gemäß einem der Ansprüche 1 bis 5, **dadurch gekennzeichnet, dass** die ethylenisch ungesättigte Carbonsäure, deren Salz oder Ester eine natürlich vorkommende ethylenisch ungesättigte Carbonsäure, deren Salz oder Ester ist und im Gemisch mit weiteren natürlich vorkommenden Carbonsäuren, deren Salzen oder Estern verwendet wird, wobei der Anteil der ethylenisch ungesättigte Carbonsäure, deren Salz oder Ester im Gemisch mindestens 30 Gew.-% beträgt.

7. Verfahren gemäß einem der Ansprüche 1 bis 6, **dadurch gekennzeichnet, dass** der Oberflächennachvernetzer das Epoxidierungsprodukt einer ethylenisch ungesättigten Carbonsäure, deren Salz oder Ester ist.

8. Verfahren gemäß einem der Ansprüche 1 bis 6, **dadurch gekennzeichnet, dass** der Ester der ethylenisch ungesättigten Carbonsäure ein pflanzliches und/oder tierisches Öl ist.

9. Verfahren gemäß einem der Ansprüche 1 bis 6, **dadurch gekennzeichnet, dass** der Ester der ethylenisch ungesättigten Carbonsäure ein pflanzliches und/oder tierisches Öl ist, wobei der Anteil der ethylenisch ungesättigten Carbonsäure mindestens 30 Gew.-% beträgt, bezogen auf die Gesamtmenge an Carbonsäuren im pflanzlichen und/oder tierischen Öl.

10. Verfahren gemäß Anspruch 8 oder 9, **dadurch gekennzeichnet, dass** das pflanzliche Öl Leinöl und/oder Vernoniaöl ist.

11. Verfahren gemäß einem der Ansprüche 1 bis 10, **dadurch gekennzeichnet, dass** die thermische Oberflächennachvernetzung bei 50 bis 150°C in Gegenwart von Sauerstoff durchgeführt wird.

12. Verfahren gemäß einem der Ansprüche 1 bis 6, **dadurch gekennzeichnet, dass** der Oberflächennachvernetzer ein epoxidiertes pflanzliches und/oder tierisches Öl ist.

13. Verfahren gemäß einem der Ansprüche 1 bis 6, **dadurch gekennzeichnet, dass** der Oberfiächennachvernetzer ein epoxidiertes pflanzliches und/oder tierisches Öl ist, wobei der Anteil der ethylenisch ungesättigten Carbonsäure mindestens 30 Gew.-% beträgt, bezogen auf die Gesamtmenge an Carbonsäuren im pflanzlichen und/oder tierischen Öl.

14. Verfahren gemäß Anspruch 12 oder 13, **dadurch gekennzeichnet, dass** das epoxidierte pflanzliche Öl epoxidiertes Leinöl ist.

15. Verfahren gemäß einem der Ansprüche 1 bis 14, **dadurch gekennzeichnet, dass** die wasserabsorbierenden Polymerpartikel eine Zentrifugenretentionskapazität von mindestens 15 g/g aufweisen.

16. Wasserabsorbierende Polymerpartikel, erhältlich gemäß einem Verfahren der Ansprüche 1 bis 15.

17. Hygieneartikel, enthaltend wasserabsorbierende Polymerpartikel gemäß Anspruch 16.

## Claims

1. A process for surface postcrosslinking water-absorbing polymer particles which are obtained by polymerizing a monomer solution or suspension comprising
a) at least one ethylenically unsaturated monomer bearing acid groups,
b) at least one crosslinker,
c) at least one initiator,
d) optionally one or more ethylenically unsaturated monomers copolymerizable with the monomers mentioned under a) and
e) optionally one or more water-soluble polymers,
at least one surface postcrosslinker is applied to the water-absorbing polymer particles and the water-absorbing polymer particles are thermally surface postcrosslinked, wherein the surface postcrosslinker is at least one ethylenically unsaturated carboxylic acid having at least 10 carbon atoms and at least two carbon-carbon double bonds, or a salt, ester and/or epoxidation product thereof, and the amount of ethylenically unsaturated carboxylic acid having at least 10 carbon atoms and at least two carbon-carbon double bonds, or a salt, ester and/or epoxidation product thereof, based on the water-absorbing polymer particles, is from 0.02 to 2% by weight.

2. The process according to claim 1, wherein the ethylenically unsaturated carboxylic acid or the salt or ester thereof has at least two adjacent carbon-carbon double bonds.

3. The process according to either of claims 1 and 2, wherein the ethylenically unsaturated carboxylic acid or the salt or ester thereof, in addition to the carbon-carbon double bond, has at least one epoxy group.

4. The process according to any one of claims 1 to 3, wherein the ethylenically unsaturated carboxylic acid or the salt or ester thereof additionally has at least one epoxy group adjacent to the carbon-carbon double bond

5. The process according to any one of claims 1 to 4, wherein the ethylenically unsaturated carboxylic acid or the salt or ester thereof is linolenic acid or a salt or ester thereof and/or vernolic acid or a salt or ester thereof.

6. The process according to any one of claims 1 to 5, wherein the ethylenically unsaturated carboxylic acid or the salt or ester thereof is a naturally occurring ethylenically unsaturated carboxylic acid or a salt or ester thereof and is used in a mixture with further naturally occurring carboxylic acids or salts or esters thereof, where the proportion of the ethylenically unsaturated carboxylic acid or salt or ester thereof in the mixture is at least 30% by weight.

7. The process according to any one of claims 1 to 6, wherein the surface postcrosslinker is the epoxidation product of an ethylenically unsaturated carboxylic acid or salt or ester thereof.

8. The process according to any one of claims 1 to 6, wherein the ester of the ethylenically unsaturated carboxylic acid is a vegetable and/or animal oil.

9. The process according to any one of claims 1 to 6, wherein the ester of the ethylenically unsaturated carboxylic acid is a vegetable and/or animal oil, where the proportion of the ethylenically unsaturated carboxylic acid is at least 30% by weight based on the total amount of carboxylic acids in the vegetable and/or animal oil.

10. The process according to claim 8 or 9, wherein the vegetable oil is linseed oil and/or vernonia oil.

11. The process according to any one of claims 1 to 10, wherein the thermal surface postcrosslinking is performed at from 50 to 150°C in the presence of oxygen.

12. The process according to any one of claims 1 to 6, wherein the surface postcrosslinker is an epoxidized vegetable and/or animal oil.

13. The process according to any one of claims 1 to 6, wherein the surface postcrosslinker is an epoxidized vegetable and/or animal oil, where the proportion of the ethylenically unsaturated carboxylic acid is at least 30% by weight based on the total amount of carboxylic acids in the vegetable and/or animal oil.

14. The process according to claim 12 or 13, wherein the epoxidized vegetable oil is epoxidized linseed oil.

15. The process according to any one of claims 1 to 14, wherein the water-absorbing polymer particles have a centrifuge retention capacity of at least 15 g/g.

16. Water-absorbing polymer particles obtainable by a process of claims 1 to 15.

17. A hygiene article comprising water-absorbing polymer particles according to claim 16.

## Revendications

1. Procédé pour la post-réticulation superficielle de particules de polymère absorbant l'eau, les particules de polymère absorbant l'eau étant obtenues par polymérisation d'une solution ou suspension de monomères, contenant
a) au moins un monomère à insaturation éthylénique, contenant des groupes acides,
b) au moins un agent de réticulation,
c) au moins un amorceur,
d) en option un ou plusieurs monomères à insaturation éthylénique, copolymérisables avec les monomères nommés en a),
e) en option un ou plusieurs polymères hydrosolubles,
au moins un agent de post-réticulation superficielle étant appliqué sur les particules de polymère absorbant l'eau et les particules de polymère absorbant l'eau étant post-réticulées thermiquement en surface, **caractérisé en ce que** l'agent de post-réticulation superficielle est au moins un acide carboxylique à insaturation éthylénique ayant au moins 10 atomes de carbone et comportant au moins deux doubles liaisons carbone-carbone, son sel, ester et/ou produit d'époxydation et la quantité d'acide carboxylique à insaturation éthylénique ayant au moins 10 atomes de carbone et comportant au moins deux doubles liaisons carbone-carbone, de son sel, ester et/ou produit d'époxydation, par rapport aux particules de polymère absorbant l'eau, vaut de 0,02 à 2 % en poids.

2. Procédé selon la revendication 1, **caractérisé en ce que** l'acide carboxylique à insaturation éthylénique, son sel ou ester comporte au moins deux doubles liaisons carbone-carbone contiguës.

3. Procédé selon l'une quelconque des revendications 1 et 2, **caractérisé en ce que** l'acide carboxylique à insaturation éthylénique, son sel ou ester comporte, en plus de la double liaison carbone-carbone, au moins un groupe époxy.

4. Procédé selon l'une quelconque des revendications 1 à 3, **caractérisé en ce que** l'acide carboxylique à insaturation éthylénique, son sel ou ester comporte en outre au moins un groupe époxyde contigu à la double liaison carbone-carbone.

5. Procédé selon l'une quelconque des revendications 1 à 4, **caractérisé en ce que** l'acide carboxylique à insaturation éthylénique, son sel ou ester est l'acide linolénique, son sel ou ester et/ou l'acide vernolique, son sel ou ester.

6. Procédé selon l'une quelconque des revendications 1 à 5, **caractérisé en ce que** l'acide carboxylique à insaturation éthylénique, son sel ou ester est un acide carboxylique à insaturation éthylénique existant dans la nature, son sel ou ester et est utilisé en mélange avec d'autres acides carboxyliques existant dans la nature, leurs sels ou esters, la proportion de l'acide carboxylique à insaturation éthylénique, de son sel ou ester dans le mélange étant d'au moins 30 % en poids.

7. Procédé selon l'une quelconque des revendications 1 à 6, **caractérisé en ce que** l'agent de post-réticulation superficielle est le produit d'époxydation d'un acide carboxylique à insaturation éthylénique, de son sel ou ester.

8. Procédé selon l'une quelconque des revendications 1 à 6, **caractérisé en ce que** l'ester de l'acide carboxylique à insaturation éthylénique est une huile végétale et/ou animale.

9. Procédé selon l'une quelconque des revendications 1 à 6, **caractérisé en ce que** l'ester de l'acide carboxylique à insaturation éthylénique est une huile végétale et/ou animale, la proportion de l'acide carboxylique à insaturation éthylénique étant d'au moins 30 % en poids, par rapport à la quantité totale d'acides carboxyliques dans l'huile végétale et/ou animale.

10. Procédé selon la revendication 8 ou 9, **caractérisé en ce que** l'huile végétale est l'huile de lin et/ou l'huile de vernonia.

11. Procédé selon l'une quelconque des revendications 1 à 10, **caractérisé en ce que** la post-réticulation superficielle thermique est effectuée à une température de 50 à 150 °C en présence d'oxygène.

12. Procédé selon l'une quelconque des revendications 1 à 6, **caractérisé en ce que** l'agent de post-réticulation superficielle est une huile végétale et/ou animale époxydée.

13. Procédé selon l'une quelconque des revendications 1 à 6, **caractérisé en ce que** l'agent de post-réticulation superficielle est une huile végétale et/ou animale époxydée, la proportion de l'acide carboxylique à insaturation éthylénique étant d'au moins 30 % en poids, par rapport à la quantité totale d'acides carboxyliques dans l'huile végétale et/ou animale.

14. Procédé selon la revendication 12 ou 13, **caractérisé en ce que** l'huile végétale époxydée est l'huile de lin époxydée.

15. Procédé selon l'une quelconque des revendications 1 à 14, **caractérisé en ce que** les particules de polymère absorbant l'eau présentent une capacité de rétention centrifuge d'au moins 15 g/g.

16. Particule de polymère absorbant l'eau, pouvant être obtenue conformément à un procédé selon les revendications 1 à 15.

17. Article d'hygiène, contenant des particules de polymère absorbant l'eau selon la revendication 16.
